# EUROPÄISCHE PATENTSCHRIFT

(11) **EP 3 019 868 B1**
(45) Veröffentlichungstag und Bekanntmachung des Hinweises auf die Patenterteilung: **15.01.2020**
(21) Anmeldenummer: 14747851.5
(22) Anmeldetag: 07.07.2014
(51) Int. Cl.: G01N 33/558, G01N 33/543, C12Q 1/6823, G01N 33/58

(54) **UNIVERSELLES VERFAHREN ZUR DETEKTION VON DIVERSEN ANALYTEN**
UNIVERSAL METHOD FOR DETECTING VARIOUS ANALYTES
PROCÉDÉ UNIVERSEL DE DÉTECTION DE DIVERS ANALYTES

(30) Priorität: 06.07.2013 DE 102013213279
(43) Veröffentlichungstag der Anmeldung: 18.05.2016
(73) Patentinhaber: AJ Innuscreen GmbH, 13125 Berlin (DE); Moldiax GmbH, 07745 Jena (DE)
(72) Erfinder: GRASER, Elmara, 13088 Berlin (DE); HILLEBRAND, Timo, 15366 Hoppegarten (DE)
(74) Vertreter: Wehlan, Martin
(86) Internationale Anmeldenummer: PCT/EP2014/064444
(87) Internationale Veröffentlichungsnummer: WO 2015/004057

(56) Entgegenhaltungen:
- WO-A2-2007/133704
- US-A1- 2009 005 255
- BYUNG-KEUN OH ET AL: "A Fluorophore-Based Bio-Barcode Amplification Assay for Proteins", SMALL, Bd. 2, Nr. 1, 1. Januar 2006 (2006-01-01), Seiten 103-108, XP055149853, ISSN: 1613-6810, DOI: 10.1002/smll.200500260
- DIETMAR KNOPP ET AL: "Review: Bioanalytical applications of biomolecule-functionalized nanometer-sized doped silica particles", ANALYTICA CHIMICA ACTA, Bd. 647, Nr. 1, 1. August 2009 (2009-08-01), Seiten 14-30, XP055150144, ISSN: 0003-2670, DOI: 10.1016/j.aca.2009.05.037
- NAM JWA-MIN ET AL: "Detection of proteins using a colorimetric bio-barcode assay", 1 January 2007 (2007-01-01), NATURE PROTOCOLS, NATURE PUBLISHING GROUP, GB, PAGE(S) 1438 - 1444, XP001539928, ISSN: 1750-2799

## Beschreibung

Die vorliegende Erfindung betrifft ein multiplexfähiges Verfahren zur Verstärkung eines Signals für die Detektion unterschiedlichster Analyten für medizinische, biologische und biotechnologische Bereiche. Die Gestaltung des Verfahrens erlaubt es, eine Detektion des Analyten sowohl geräteunabhängig als auch voll automatisiert durchzuführen.

### Stand der Technik

Sowohl die molekularbiologische Diagnostik als auch die Analyse und der Nachweis von Nukleinsäuren, Proteinen, Antikörpern, immunogenen Substanzen etc. ist zu einem unverzichtbaren Bestandteil der modernen medizinischen Labordiagnostik, der forensischen Diagnostik, der veterinärmedizinischen Labordiagnostik oder der Lebensmittel- und Umweltdiagnostik geworden.

Revolutioniert wurde die genetische Diagnostik mit der Erfindung der PCR-Technologie (US 4,683,202), die es gestattet, jede beliebige Nukleinsäuresequenz spezifisch zu vervielfachen. Einen weiteren wesentlichen Vorteil brachte die Einführung von real-time PCR-Anwendungen (US 5,210,015, US 5,716,784 und US 5,487,972). Darüber hinaus gibt es weitere enzymatische Amplifizierungsverfahren, die bei konstanter Temperatur durchgeführt werden, wie NASBA oder Qß- Replikase Assays oder andere isothermale Techniken (J.Compton Nature 350; 91-92; 1991, WO8706270, EP 1724362 A1 etc.) und die für dieselbe Zielstellung eingesetzt werden. Weitere Nachweistechnologien sind u.a. ELISA's zum Nachweis von Proteinen oder Nukleinsäuren (R. Yalow et al. J. Clin. Invest.. 39; 1157-75; 1960, Blotting-Techniken zum Nachweis von Proteinen und Nukleinsäuren Southern (E.M. J. Mol. Biol. 98 ; 503-517 ; 1975, Alwine JC et al. Proc. Natl. Acad. Sci. U. S. A 74 ; 5350-5354 ; 1977, Renart, J. et al. Proc. Natl. Acad. Sci. U. S. A. 7; 3116-3120; 1979), Biochip- Techniken zum Nachweis von Proteinen und Nukleinsäuren (z.B. WO 8808875), Lateral Flow Teststreifen zum Nachweis von Proteinen und Nukleinsäuren (z.B. US 4956302) oder der Nachweis mittels Biobarcodes für Proteine und Nukleinsäuren (US 20030054358 A1). Viele dieser Nachweismethoden benötigen komplexe Reagenzienmixturen incl. unterschiedlicher Enzyme für die Verstärkung von Signalen für den Nachweis von Analyten (PCR, real time PCR, NASBA; ELISA's etc.). Darüber hinaus sind eine Reihe dieser Techniken nur für den Einsatz zum spezifischen Nachweis von Nukleinsäuren geeignet und können nicht für den Nachweis von Proteinen eingesetzt werden. Der Nachweis von Proteinen in Bezug auf die Nachweisempfindlichkeit ist deutlich begrenzter als der Nachweis von Nukleinsäuren mittels der aufgeführten Techniken wie PCR oder real time PCR.

Viele dieser Verfahren sind darüber hinaus an teure gerätetechnische Systeme gekoppelt (PCR, real time PCR, Biobarcodes) oder verlangen eine aufwendige Manipulation mit den Nachweisstoffen (ELISA, Blotting, Chip).

Eine universelle Möglichkeit des Nachweises für beliebige Analyten (Proteine, Nukleinsäuren, etc.) ist mit der Biobarcode- Technologie gegeben (Nature Protocols; Vol. 1, No. 1; 2006).

Für den Nachweis von Nukleinsäuren basiert diese Technologie auf einer primären Hybridisierungsreaktion einer Zielnukleinsäure (fragmentierte DNA oder RNA) an ein Fängeroligonukleotid. Dieses Fängeroligonukleotid befindet sich kovalent gebunden an einem magnetischen Partikel. Nach der Hybridisierung der Zielnukleinsäure mit dem Fängeroligonukleotid erfolgt die magnetische Separation der Partikel und Waschschritte zur Entfernung von nichtgebundenen Nukleinsäuren. In einer zweiten Reaktionsstufe erfolgt die Zugabe von Goldpartikeln, welche ein Oligonukleotid enthalten, welches wiederum an die Fängernukleinsäure des magnetischen Partikels bereits gebundene Targetnukleinsäure hybridisieren kann. Desweiteren enthalten die Goldpartikel eine sog. Biobarcode DNA und werden deshalb auch als Nachweispartikel bezeichnet. Nach erneuter Separation des Komplexes Magnetpartikel-Targetnukleinsäure-Nachweispartikel und weiteren Waschschritten erfolgt die Nachweisreaktion. Dazu wird die Biobarcode DNA freigesetzt. Dies erfolgt z.B. bei einer Ausführungsvariante, bei welcher die Biobarcode DNA über Thiolgruppen an den Goldpartikeln gebunden ist, durch die Zugabe von DTT. Die freigesetzte Biobarcode DNA wird dann in weiteren Schritten an einer festen Phase, z.B. an einen Chip mit einem partiell zur Biobarcode DNA komplementären Fängeroligonukleotid hybridisiert. Nachfolgend erfolgt die Zugabe von Goldpartikeln, die ebenfalls ein partiell zur Biobarcode DNA komplementäres Oligonukleotid tragen. Dieses Sandwich wird dann durch Zugabe von Silberionen mittels eines Scanners detektiert.

Der Nachweis von Proteinen erfolgt analog. Dabei ist das Magnetpartikel mit einem primären Antikörper konjugiert, welcher das nachzuweisende Protein erkennt. Die Nachweispartikel (Goldpartikel versehen mit hunderten Biobarcode DNA's) tragen den sekundären Antikörper. Es bildet sich wiederum ein Komplex (Magnetpartikel mit primärem Antikörper-Protein-Goldpartikel mit Biobarcode DNA und sekundärem Antikörper.

Es folgen wieder magnetische Separation und Waschschritte. Die Detektion erfolgt wie beim Nachweis von Nukleinsäuren über die Freisetzung der Biobarcode DNA auf einem Array.

Diese Technologie ist kommerziell verfügbar und an spezifische Gerätesysteme gebunden (http://www.nanosphere.us/page/direct-detection-nucleic-acids).

Diese Tests sind teuer und sowohl an die Gerätesysteme für die Enddetektion als auch an die Benutzung der Chemikalien (DTT) zur Ablösung der Barcodes gebunden. Darüber hinaus benötigt das System eine spezielle Nachweistechnologie in Form der Hybridisierung der freigesetzten Biobarcode DNA mit einem Fängeroligonukleotid sowie mit Goldpartikeln und mit nachfolgender Silberverstärkung, was das Verfahren zu einem aufwendigen Vielschritt- System macht.

Ein weiterentwickeltes Verfahren, welches einige Prinzipien der Biobarcode Methode nutzt, basiert darauf, dass die markierten Barcode - Moleküle bereits mit einer nachzuweisenden Fluoreszenzmarkierung vorliegen. Diese Nachweismoleküle sind spezifisch über eine Thiolgruppe an Goldpartikel gebunden. Die Ablösung der Nachweispartikel erfolgt wieder über die Zugabe von DTT. Damit ist dieses System an Goldpartikel gebunden. Andere Partikelarten sind nicht möglich. Darüber hinaus bedarf der Nachweis eines Fluoreszenzsignals einer sehr empfindlichen und teuren Messtechnik und kann somit nicht für einfache point of care Applikationen eingesetzt werden (D. Zhang et al. Biosensors and Bioelectronics 24 (2009) 1377-1381).

In der Offenlegungsschrift US 2009/0005255 A1 wird ein Verfahren beschrieben, welches keine Goldpartikel, sondern spezifische Polymerpartikel nutzt. Diese Polymerpartikel übernehmen die Funktion der Goldpartikel als Nachweispartikel. An den Polymerpartikeln befinden sich kovalent gebundene DNA-Moleküle. Die Barcode-Moleküle, die für die Nachweisreaktion benutzt werden, befinden sich an den kovalent gebundenen DNA-Molekülen (sind an diese Moleküle hybridisiert). An diese Barcode-Molekülen sind weitere Nanopartikel gekoppelt. Diese Nanopartikel enthalten einen Magnetitkern. Die Detektionsreaktion erfolgt über eine komplizierte Messmethodik zum Nachweis einer Magnetresonanz. Damit ist dieses Verfahren ebenfalls aufwendig und an eine komplizierte Messtechnik und Apparatur gebunden.

In einer weiteren Druckschrift (US 2008/0268450 A1) ist ein Verfahren offenbart, welches sich auf einen antikörpervermittelten Nachweis begrenzt. Anstelle von Goldpartikeln werden Silikapartikel eingesetzt, welche aminomodifizierte Oberflächen aufweisen. Das Nachweisverfahren (auch ein multiplexes) basiert auf sequenzspezifischen Nachweisreaktionen der Barcodes, wobei auch dieses Verfahren mehrere umfangreiche und zeitaufwendige Arbeitsschritte benötigt. Der finale Nachweis erfolgt über ein Scanning Elektronenmikroskop auf einem TLC-Chip. Damit ist auch dieses Verfahren aufwendig und an eine teure Gerätekomponente gebunden.

Die US-Patentanmeldung US2006/0040286 A1 beschreibt ebenfalls ein Biobarcode-Verfahren zum Nachweis von Analyten. In diesem Patent werden wieder Goldpartikel eingesetzt. Die Barcodes müssen über ihre spezifische Sequenz detektiert werden. Dieses macht das Verfahren aufwendig und störanfällig. Die finale Nachweisreaktion erfolgt wieder auf einer Chipoberfläche über diverse Schritte, incl. einer Silberabscheidung oder Fluoreszenzmessung. Damit ist auch dieses Verfahren zwingend an ein Nachweisgerät gebunden und darüber hinaus zeit- und arbeitsintensiv.

Die Offenlegungsschrift WO 2007/133704 A2 beschreibt ein Biobarcode-Verfahren zur Detektion von biologischen Materialien. Nicht-Nukleinsäure-Marker oder Reporter werden als biochemische Barcodes zum Nachweis mindestens eines bestimmten Zielanalyten in einer Lösung verwendet. Die Nachweismoleküle dienen aber nicht gleichzeitig dem Nachweis des Analyten und der Detektion, weil der Zielanalyt nicht über die Reporter an die Nanopartikel gebunden ist, sondern über einen Antikörper (siehe WO 2007/133704 A2 - Figur 1). Deshalb kann dieses Nachweisverfahren nicht auf einem Lateral-Flow-Streifen erfolgen.

### Aufgabe der Erfindung

Die Aufgabe der vorliegenden Erfindung definiert sich aus den aufgeführten Nachteilen von bekannten Nachweissystemen. Insbesondere soll das erfindungsgemäße Verfahren einfach in der Durchführung sein. Der zeitliche Aufwand soll gering sein. Darüber hinaus soll die Nachweisreaktion auch ohne teurere Gerätesysteme funktionieren und sich nicht extrem aufwendiger Messprinzipien bedienen müssen. Trotzdem soll das erfindungsgemäße Verfahren eine Multiplexfähigkeit und eine ausreichende Sensitivität vorweisen.

### Lösung der Aufgabe

Die Aufgabe wurde gemäß den Merkmalen der Patentansprüche gelöst.

Das erfindungsgemäße Verfahren ermöglicht eine schnelle Signalgenerierung ohne komplizierte Gerätetechnik, eine sehr hohe Empfindlichkeit und ist darüber hinaus gleichermaßen sowohl für den Nachweis von Nukleinsäuren als auch für den Nachweis von Proteinen oder anderen immunogenen Analyten geeignet. Die Detektion kann nach wenigen Schritten auf einem einfachen Teststreifen erfolgen. Die Auswertung erfolgt optisch mit bloßem Auge und benötigt keine aufwendigen sekundären Nachweisreaktionen. Das Verfahren ist darüber hinaus vollautomatisierbar, multiplexfähig und kann als ein geschlossenes, kontaminationsfreies System eingesetzt werden.

Das erfindungsgemäße Verfahren zum Nachweis eines Analyten nutzt ebenfalls zwei spezifische Partikel - "Separationspartikel" ("SP") sowie Nachweispartikel ("NP"). Dabei können beide Partikel- Arten verschiedene Formen und Größen aufweisen, vorzugsweise Größen zwischen 1 nm und 10 µm. Dabei muss es sich bei den Nachweispartikeln nicht um Goldpartikel handeln.

Bei den Separationspartikeln handelt es sich um magnetische oder paramagnetische Partikel (magnetische Separation) oder um Partikel mit einer spezifischen Funktionalität, die einen Separationsprozess ermöglicht. Die Separationspartikel tragen auf ihrer Oberfläche Moleküle funktionale Gruppen, die in spezifische Wechselwirkung mit dem nachzuweisenden Analyten treten können (Fängermoleküle). Diese Partikel können ggf. auch durch eine feste Phase ersetzt werden, die mit den gleichen funktionalen Gruppen beschichtet ist.

Die Nachweispartikel können aus unterschiedlichsten Materialien wie Gold, Latex, Polymeren oder Fullerenen etc. bestehen. Sie besitzen keine Separationsfunktionalitäten. Nachweispartikel tragen auf ihrer Oberfläche ebenfalls Moleküle oder funktionale Gruppen (Fängermoleküle), welche in spezifische Wechselwirkung mit dem nachzuweisenden Analyten treten können, wobei diese Moleküle oder funktionale Gruppen eine andere bzw. gleiche (im Fall von mehreren Bindungsepitopen gleicher Art) Zielregion (DNA- Sequenz, Epitop, Proteinsequenz, etc.) des nachzuweisenden Analyten erkennen. Weiterhin tragen die Nachweispartikel sog. Nachweismoleküle, wie dies auch im Falle der Biobarcode DNA ist. Die Nachweismoleküle können auch durch die Nachweispartikel eingeschlossen werden.

Diese Nachweismoleküle dienen im Detektionsprozess dazu, ein Detektionssignal zu generieren. Damit dies möglich ist, ist die Kopplung der Nachweismoleküle an den Nachweispartikel der Art, dass es sich um eine reversible Kopplung handelt, d.h. die Nachweismoleküle können wieder vom Nachweispartikel getrennt werden. Dies kann chemisch, physikalisch oder enzymatisch erfolgen oder in Kombination dieser Varianten.

Durch die vorliegenden Fängermoleküle auf den Separationspartikeln und den Nachweispartikeln ist es möglich, dass beide Partikel hochspezifisch mit einem Analyten eine Bindung eingehen können. Um die Bindung mehrerer Analyten an ein und denselben Nachweispartikel zu vermeiden, ist es zweckmäßig, zunächst die Separationspartikel mit dem Analyten zu verbinden und die Nachweispartikel erst nachfolgend der Reaktion zuzuführen. Eine gleichzeitige Zugabe der Partikel beider Arten zu den Analyten ist jedoch auch ebenfalls möglich.

Dabei kann die Bindung kovalenter Natur sein, es kann sich um Wasserstoffbrücken handeln, es kann sich um Antigen- Antikörper- Bindungen handeln etc..

Durch diese Wechselwirkung mit dem Analyten erfolgt die Verbindung zwischen den Separationspartikeln und den Nachweispartikeln mit dem Analyten, die eine Separation der gebundenen Nachweispartikel von den ungebundenen Nachweispartikeln ermöglicht. Also nur in dem Fall, dass der Analyt in der Partikelmischung vorhanden ist, können die Nachweispartikel zusammen mit den Separationspartikeln separiert werden. Im anderen Fall verbleiben sie in der Lösung.

Die Separation erfolgt auf eine von den Separationsqualitäten der Separationspartikel bzw. Separationsoberfläche abhängige Art (z.B. durch Magnetseparation der Magnetpartikel).

Wie bei der Biobarcode-Methode beginnt die Detektionsreaktion mit der Freisetzung der Nachweismoleküle vom Nachweispartikel. Der erfinderische Vorteil des Verfahrens offenbart sich wie folgt: Bei den Nachweismolekülen handelt es sich im Gegensatz zur Biobarcode-Technologie vorzugsweise um einen oder mehrere DNA-Stränge, welche an das mit Partikeln kovalent gebundene DNA-Fragment reversibel gekoppelt sind (im einfachsten Fall durch Hybridisierung). Im Gegensatz zu allen bekannten Methoden, welche auf dem Prinzip der Biobarcode-Methode basieren, sind die Nachweismoleküle durch eine erfindungsgemäße Andersartigkeit charakterisiert. Diese DNA-Stränge, welche für die finale Nachweisreaktion verantwortlich sind, sind erfindungsgemäß mit jeweils mindestens zwei Labeln verknüpft, von denen mindestens ein Label immunogen ist und ggf. ein Label eine direkte oder indirekte Detektionsreaktion ermöglicht. Dadurch dass die Nachweismoleküle mit zwei Labeln versehen sind, ist es möglich, die Nachweismoleküle über ein Label an eine feste Phase zu binden, wobei das andere Label dann direkt oder indirekt für die Detektion genutzt wird. Damit ist im Unterschied zu den aufgeführten anderen Biobarcode-basierten Verfahren keine weitere Manipulation der freigesetzten Nachweismoleküle (z.B. Hybridisierung etc.) notwendig, was das Verfahren deutlich vereinfacht.

Durch die Möglichkeit, mehrere Nachweis-DNA Stränge an einem kovalent an Partikel gebundenen DNA-Strang reversibel zu koppeln, kann die Sensitivität des Systems noch erhöht werden, da für die Nachweisreaktion mehr markierte DNA zur Verfügung steht. Die Nachweisreaktion basiert auf der Ablösung der reversibel gebundenen markierten DNA-Stränge extrem einfach und schnell und bedarf keiner weiteren Gerätetechnik. Sie erfolgt z.B. dadurch, dass eine doppelendständig (z.B. mit FITC und Biotin) markierte DNA auf einen Teststreifen (Lateral Flow Streifen) überführt wird. Dieser Teststreifen enthält zu den beiden Markierungen komplementäre Moleküle. Der Nachweis erfolgt durch eine nachfolgend sichtbar werdende Testlinie auf dem Streifen unter Nutzung der bekannten Lateral Flow Techniken.

Wenn eine Fluoreszenzmessung erwünscht wird, kann die DNA mit einem oder mehreren Fluorophoren markiert werden. Es können auch chemische Markierungsgruppen, die zu einer Milieuveränderung führen, oder Enzyme eingesetzt werden.

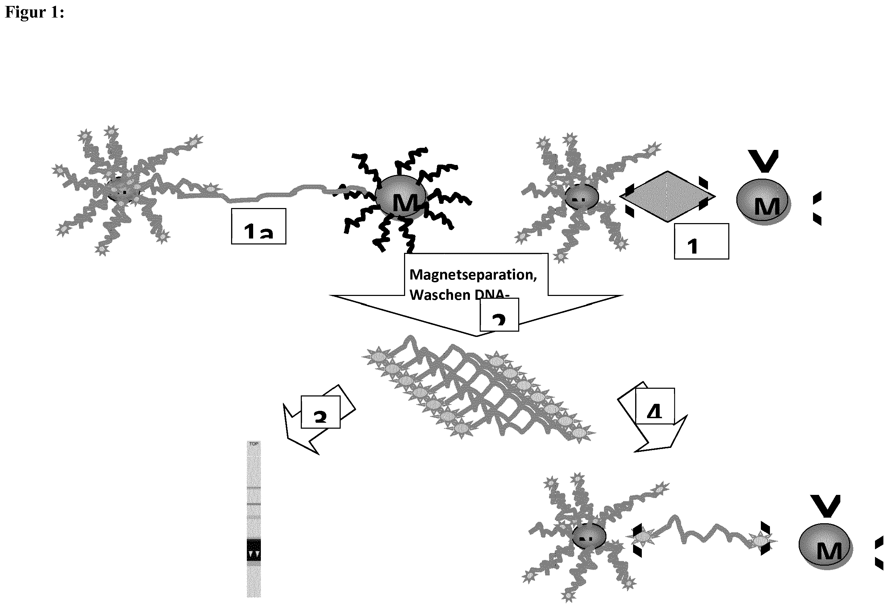

Nach Freisetzung der Nachweismoleküle kann der Nachweis der vorangegangenen Reaktion innerhalb von wenigen Minuten erfolgen. Es ist keine aufwendige Prozessierung und Detektionsgeräte wie im Falle der Biobarcode-Nachweisreaktion notwendig. Das erfindungsgemäße Verfahren ist unter der Figur 1 schematisch dargestellt.

Wie schon beschrieben, wird bei der Biobarcode-Methode die freigesetzte Biobarcode DNA in weiteren Schritten an einer festen Phase mit einem partiell zur Biobarcode-DNA komplementären Fängeroligonukleotid hybridisiert. Nachfolgend erfolgt die Zugabe von Goldpartikeln, die ebenfalls ein partiell zur Biobarcode DNA komplementäres Oligonukleotid tragen. Dieses Sandwich wird dann durch Zugabe von Silberionen mittels eines Scanners detektiert. Die in der Schrift aufgeführten alternativen Varianten der Biobarcode-Technik, bedienen sich ebenfalls sehr komplizierten Messtechniken und bedürfen teurer Gerätesysteme. Kein Verfahren kann auf einem klassischen Lateral Flow Teststreifen ausgelesen werden.

Als Nachweismoleküle können nicht nur DNAs eingesetzt, sondern prinzipiell jedes markierbare Molekül kann als Nachweismolekül dienen. Vorzugsweise sind zwei Markierungen am Nachweismolekül vorhanden, wobei eine immunogen ist.

Es ist nicht nur möglich, dass sich die Nachweismoleküle auf der Oberfläche von Partikeln befinden, sondern die Nachweismoleküle können auch durch (vorzugsweise) Polymerpartikel eingeschlossen sein, also verpackt vorliegen. Die Freisetzung erfolgt dann durch Änderung der physikalischen oder chemischen Bedingungen (z.B. pH-Wert, Temperatur).

Der Nachweis der freigesetzten Nachweismoleküle kann - im Fall einer DNA - auch über eine enzymatische Amplifizierungsreaktion, PCR, real time PCR oder andere isothermale Techniken erfolgen.

Zur Erhöhung der Sensitivität ist eine Kaskadierung der Reaktion möglich.

Es ist erfindungsgemäß möglich, dass silikatische Partikel verwendet werden, an denen die Nachweismoleküle via Spacer gebunden sind. Es ist auch möglich, dass magnetische Partikel als Separationspartikel mit magnetischem Kern aber silikatischer Hülle eingesetzt werden.

Ein weiterer Vorteil der Erfindung ist, Partikel einzusetzen, die keinen spezifischen Wechselwirkungspartner auf der Oberfläche tragen und unspezifisch Analyten binden. Die Bindung kann dann entweder adsorptiv erfolgen oder aber über Substanzen erfolgen, die mehrere Substanzen binden können.

Damit wird offensichtlich, dass das erfindungsgemäße Verfahren deutlich einfacher in seiner Durchführung ist.

Es wird auch kein Gerät für den Nachweis notwendig sein. Die Testdurchführung qualifiziert das erfindungsgemäße Verfahren damit auch in idealster Weise als ein potentielles point of care Testsystem. Die Multiplexfähigkeit ist durch die unterschiedliche Markierung der reversibel assoziierten Moleküle gegeben, wie es später in einem Anwendungsbeispiel gezeigt wird.

Eine weitere Ausführungsform des erfindungsgemäßen Verfahrens basiert darauf, dass es sich bei den Nachweispartikeln um Polymerpartikel handelt. Im Unterschied zur bereits beschriebenen Ausführungsform des erfindungsgemäßen Verfahrens befinden sich dabei die Nachweismoleküle nicht an der Oberfläche der Partikel. An der Oberfläche befinden sich nur die Moleküle oder funktionale Gruppen (Fängermoleküle), welche in spezifische Wechselwirkung mit dem nachzuweisenden Analyten treten können, wobei diese Moleküle oder funktionale Gruppen eine andere bzw. gleiche (im Fall von mehreren Bindungsepitopen gleicher Art) Zielregion (DNA- Sequenz, Epitop, Proteinsequenz, etc.) des nachzuweisenden Analyten erkennen. Nicht aber die eigentlichen Nachweismoleküle. Die erfindungsgemäßen doppeltmarkierten Nachweismoleküle (DNA- Stränge) sind im Polymerpartikel reversibel eingeschlossen. Dies kann durch klassische Techniken der Erzeugung von Polymerpartikeln erfolgen, wie dies für spezielle Verpackungen von pharmazeutischen Wirkstoffen in Polymerpartikel bekannt ist.

Die Nachweisreaktion basiert in dieser Ausführungsform darauf, dass die im Polymerpartikel eingeschlossenen Nachweismoleküle wieder freigesetzt werden. Dies kann durch physikalische, enzymatische oder auch chemische Verfahren erfolgen (z.B. Änderung des pH- Wertes, Temperaturerhöhung etc.). Die freigesetzten Nachweismoleküle werden dann in der schon beschriebenen Form für die Detektionsreaktion eingesetzt (z.B. mittels eines Teststreifens). Die Ausführungsform, dass die Nachweismoleküle in Polymerpartikel eingeschlossen sind und freigesetzt werden können, ermöglicht es auch, dass die Nachweismoleküle auch anders beschaffen sein können. Neben den doppelt markierten DNA- Strängen, die für einen direkten Nachweis eingesetzt werden, kann man auch eine nichtmarkierte Nukleinsäure verpacken. Nach der Freisetzungsreaktion kann diese Nukleinsäure z.B. für klassische Vervielfältigungsreaktion (z.B. PCR oder real time PCR) genutzt werden. Damit kann eine extreme Steigerung der Empfindlichkeit ermöglicht werden. Darüber hinaus können neben der DNA auch andere Moleküle verpackt und nach Freisetzung für eine Detektion genutzt werden (z.B. doppelt markierte Oligomere, die keine Nukleinsäure sind) .

Erfindungsgemäß können die freigesetzten Nachweismoleküle sowohl direkt nachgewiesen werden als auch für eine weitere Signalverstärkung als Targetmolekül dienen (Kaskadenprinzip). In diesem Fall werden die Nachweismoleküle mittels ihrer Doppelmarkierung an weitere Separation- und Nachweispartikel gebunden, die entsprechende Bindungsmittel an ihrer Oberfläche aufweisen (z.B. Anti- FITC- Antikörper und Streptavidin).

Das erfindungsgemäße Verfahren offenbart noch weitere Vorteile gegenüber der Biobarcode- Methode und ihren bekannten Weiterentwicklungen (Goluch ED, Stoeva SI, Lee JS, Shaikh KA, Mirkin CA, Liu C. Biosens Bioelectron; 2009 Apr 15; 24(8):2397-403).

In der Patentanmeldung Nr. US 20100081134A1 ist eine weitere Biobarcode basierte Methode beschrieben, wo als Barcodes auch die an die kovalent gebundenen DNA-Fragmente hybridisierten Moleküle verwendet werden. Diese sind aber nicht mit einem Label verknüpft, also unmarkiert. Diese Biobarcode DNA's sind auch nicht dafür bestimmt, an ein Nukleinsäure-Target zu binden. Daher entstehen folgende methodische Nachteile: Die Nachweispartikel sind ebenfalls nicht markiert und können nur indirekt nachgewiesen werden (Chip basiert oder über eine PCR) und die Nachweispartikel müssen mit zwei Sorten von kovalent gebundenen DNA's funktionalisiert werden - eine für die Bindung des Targets und die andere für die Bindung des Barcode- Moleküls (s. Fig. 13 A und B in US 20100081134A1).

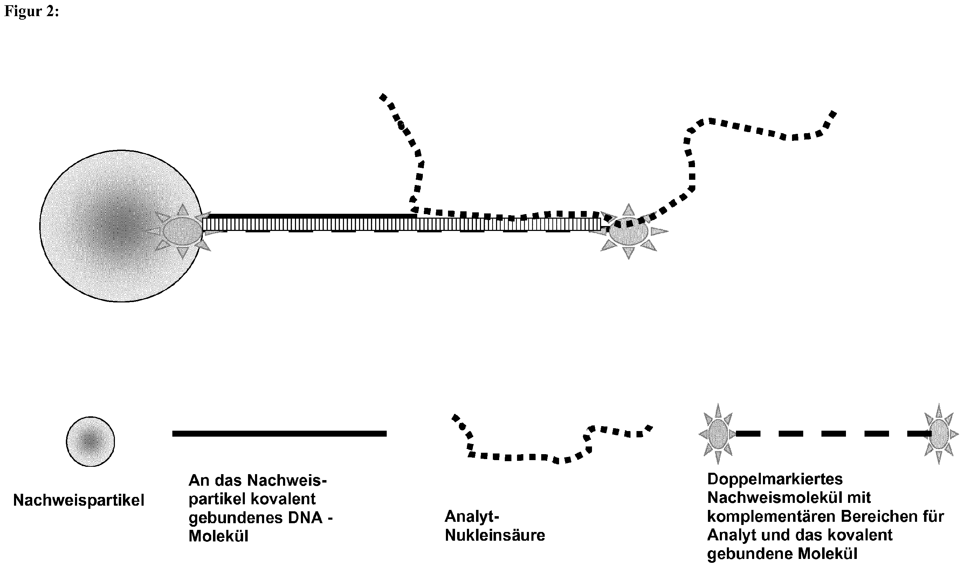

Das erfindungsgemäße Verfahren dagegen erlaubt überraschenderweise aber auch den Nachweis von Nukleinsäuren (Nachweis einer Target- DNA oder Target- RNA). Dies geschieht dadurch, dass die Nachweismoleküle die Funktion der Fängermoleküle übernehmen, indem sie partiell mit dem kovalent gebundenen DNA-Fragment hybridisieren sowie partiell auch an die nachzuweisende Targetnukleinsäure komplementär binden (Fig. 2). Dieses ist mit der beschriebenen Biobarcode Technologie nicht möglich.

Das erfindungsgemäße Verfahren offenbart noch einen weiteren wesentlichen Vorteil. Dies betrifft die reproduzierbare und kontrollierte Herstellung der eingesetzten Partikel, welche die Biobarcode DNA's enthalten. Beim Herstellungsprozess nicht herausgewaschene und nicht hybridisierte Barcode DNA's führen in der Nachweisreaktion zu falschpositiven Resultaten. Da diese DNA-Moleküle nicht markiert sind, können die Partikel nach ihrer Kopplung und den Waschschritten aber nicht auf ihre Reinheit überprüft werden. Das erfindungsgemäße Verfahren erlaubt dagegen eine effiziente Kontrolle der produzierten Partikel in Bezug auf Partikelreinheit und Partikelfunktionalität.

Eine weitere spezielle Ausführungsform des erfindungsgemäßen Verfahrens erlaubt eine kaskadierte Verstärkung der Detektionsempfindlichkeit. Diese basiert darauf, dass die an die separierten Nachweispartikel gebundenen Nachweismoleküle nach ihrer erfindungsgemäßen Ablösung oder Freisetzung als Targetmoleküle in einem zweiten Reaktionszyklus fungieren können. Die freigesetzten Nachweismoleküle werden dazu mit neuen Nachweispartikeln und neuen Separationspartikeln zusammengebracht. Diese tragen wiederum die Fängermoleküle, die mit den Nachweismolekülen reagieren. Es werden die notwendigen Bedingungen für die Bindung der Fängermoleküle beider Partikelarten an die Nachweismoleküle hergestellt (Temperatur, pH- Wert etc.). Durch diese Bindung entsteht ein Nachweispartikel-Nachweismolekül-Separationspartikel-Komplex. Die an den separierten neuen Nachweispartikeln gebundenen Nachweismoleküle werden wieder abgelöst und können jetzt wieder nachgewiesenen werden oder dienen abermals Analyten in dem nächsten Nachweiszyklus (Zyklus 3 und dann Zyklus 4 usw.). Die Wiederholung der Zyklen ermöglicht die kaskadenartige Signalverstärkung, wodurch eine enorme Sensitivität erreicht werden kann.

Der erfindungsgemäße Prozess der Detektionsmethode realisiert sich kurz zusammengefasst wie folgt (Figur 1 Beispiel - Ablauf der Methode):
1. Der Analyt wird mit Nachweispartikeln und Separationspartikeln zusammengebracht. Es werden die notwendigen Bedingungen für die spezifische Bindung der Fängermoleküle beider Partikelarten an den Analyten hergestellt (Temperatur, pH etc.). Durch diese Bindung entsteht ein Nachweispartikel-Analyt-Separationspartikel-Komplex. (Prozessdauer 10 - 30 Minuten).
2. Der oben beschriebene Komplex wird von dem an den Analyten nicht gebundenen Nachweispartikel getrennt. Diese Trennung wird mittels Bindungsfähigkeit der Separationspartikel (z.B. ihre magnetische Fähigkeiten oder speziell angebrachte Separationsmoleküle) ermöglicht. Die an die separierten Nachweispartikel gebundenen Nachweismoleküle werden abgelöst (oder im Falle, der Nutzung von in Polymerpartikel verpackter Nachweismoleküle - Freisetzung der Nachweismoleküle). Prozessdauer: 2 - 3 maliges Waschen (10 Minuten), temperaturvermittelte Ablösung oder Freisetzung von verpackten Nachweismolekülen (10 Minuten).
3. Die aus dem ersten Zyklus gewonnenen Nachweismoleküle können direkt nachgewiesen werden (Detektionszeit 10 Minuten) oder werden mit neuen Nachweispartikeln und neuen Separationspartikeln zusammengebracht. Diese tragen an sich die Fängermoleküle, die mit den Nachweismolekülen reagieren. Es werden die notwendigen Bedingungen für die Bindung der Fängermoleküle beider Partikelarten an die Nachweismoleküle hergestellt (Temperatur, pH etc.). Durch diese Bindung entsteht ein NachweispartikelNachweismolekül-Separationspartikel-Komplex.
4. Die an den separierten neuen Nachweispartikeln gebundenen Nachweismoleküle werden abgelöst. Diese dienen als Analyt in dem nächsten Nachweiszyklus (Zyklus 3 und dann Zyklus 4 usw.) bzw. können gleich detektiert werden, gerätefrei mit einem Lateral Flow Streifen.

Die Wiederholung der Zyklen ermöglicht die kaskadenartige Signalverstärkung. Gesamtarbeitsaufwand beträgt 10 - 20 Minuten oder ca. 1h incl. Inkubationszeiten.

Damit zeigt das erfindungsgemäße Verfahren eindeutige Vorteile gegenüber anderen Biobarcode-Technologie basierten Verfahren. Es ist extrem einfach und schnell in der Durchführung. Es bedarf keinerlei gerätetechnischer Ausrüstung zur Detektionsmessung. Es kann über die Kaskadierung des Reaktionsprozesses über mehrere Stufen ablaufen, was zu einer enormen Steigerung der Detektionsempfindlichkeit führt.

### Definitionen:

**Analyt bzw. Target:** nachzuweisendes Molekül, eine Gruppe an Molekülen, ein Virus, eine Zelle etc.
**Fängermoleküle:** an den Nachweispartikeln befindliche Moleküle oder ihre Gruppen, die eine spezifische Bindung (jeglicher Art, Wasserstoffbrücken, Ag- AK, Kovalent etc.) mit dem Analyten bzw. mit dem Nachweismolekül eingehen.
**Separationspartikel:** magnetische oder paramagnetische Partikel (magnetische Separation) oder Partikel mit einer spezifischen Funktionalität, die einen Separationsprozess ermöglichen. Die Separationspartikel tragen auf ihrer Oberfläche Moleküle oder funktionale Gruppen, welche in spezifische Wechselwirkung mit dem nachzuweisenden Analyten treten können (Fängermoleküle).
**Partikel:** sind Partikel von einer Größe im nm - µm- Bereich in jeglicher Form, Befassung und Material (u.a. auch Fullerene oder Polymere), welche die Fängermoleküle an ihrer Oberfläche tragen. Es sind zwei Arten von Partikeln nötig:
   1. Separations- Partikel, die mit Fängermolekülen und ggf. Separationsmolekülen beschichtet sind und in ihrer Struktur eine Möglichkeit darbieten, sie aus der Reaktionslösung zu separieren (z.B. magnetisch oder mittels Bindung der Separationsmoleküle). Diese Partikel können in dem erfindungsgemessen Verfahren auch durch eine funktionalisierte feste Phase ersetzt werden.
   2. Nachweispartikel, die mit Fängermolekülen und Nachweismolekülen beschichtet sind oder die die Nachweismoleküle verpackt haben und keine eigene Fähigkeit zur Separation besitzen.
**Separationsmoleküle:** die ggf. an der Oberfläche der Separationspartikel befindlichen Moleküle, die eine Separation dieser Partikel aus der Lösung erlauben.
**Nachweismoleküle:** sind Moleküle, die sich zunächst auf der Oberfläche der Nachweispartikel befinden, danach aber abgelöst (physikalisch oder chemisch) werden und ihrerseits als Signalmoleküle für den Endnachweis dienen. In einer weiteren Ausführungsform sind die Nachweismoleküle in Polymerpartikeln verpackt und werden für die Detektionsreaktion freigesetzt. Die Nachweismoleküle können unterschiedliche Stoffe, Polymere, Nukleinsäureketten oder einzelne Moleküle sein, die sowohl durch Fängermoleküle als auch durch Nachweismoleküle des Endnachweises erkannt werden. Vorzugsweise handelt es sich um doppeltmarkierte DNA- Moleküle, von denen mindestens ein Label immunogen ist und ein Label eine direkte oder indirekte Detektionsreaktion ermöglicht.
**Endnachweis:** kann auf unterschiedlichen Wegen erfolgen, z.B. als Lateral- Flow-Nachweis, Fluoreszenzdetektion, visueller Nachweis durch eine Farbveränderung, enzymatische Amplifizierungstechniken, elektromagnetische Messung etc. Die Nachweismoleküle und ihre Markierung werden dem Endnachweis entsprechend aufgebaut.

Die Erfindung ist nachfolgend anhand der Ausführungsbeispiele dargestellt.

### Ausführungsbeispiele

### Beispiel 1:

### Herstellung der Nachweispartikel und Überprüfung ihrer Reinheit und Funktionalität.

Als Nachweispartikel wurden COOH-funktionalisierte Polymilchsäurepartikel mit der Größe 500 nm eingesetzt. An diese Partikel wurde ein NH2- gekoppeltes Olidonukleotid (NH2- GTG TTC GTG TCA TCT AGG AG) und ein Antikörper gegen Gelbfiebervirus kovalent gebunden. Die Bindung erfolgte mit einem PolyLink- Protein Coupling Kit für COOH Mikropartikel nach Vorschriften des Kits.

Anschließend wurden die Partikel gewaschen und mit Tris- BSA geblockt.

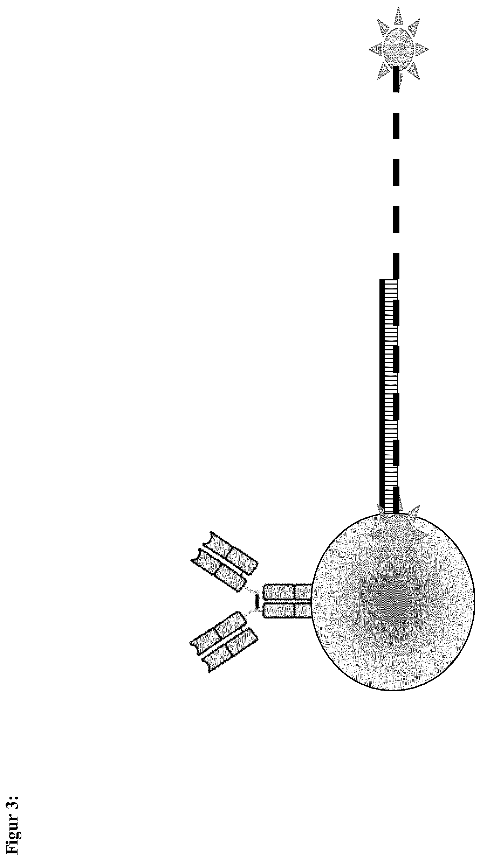

Als nächster Schritt folgte eine Hybridisierung des kovalent gebunden Oligonukleotids an das doppeltmarkierte Nachweismolekül (FITC- TGC AGC AGG TGA TAA CCT TTG G***CT CCT AGA TGA CAC GAA CAC-*** Dioxigenin). Die kursiv dargestellten Basen des Nachweismoleküls sind komplementär zu dem kovalent gebundenen Oligonukleotid. Das mit den Antikörpern und Oligonukleotiden funktionalisierte Nachweispartikel ist auf der Fig. 3 schematisch dargestellt.

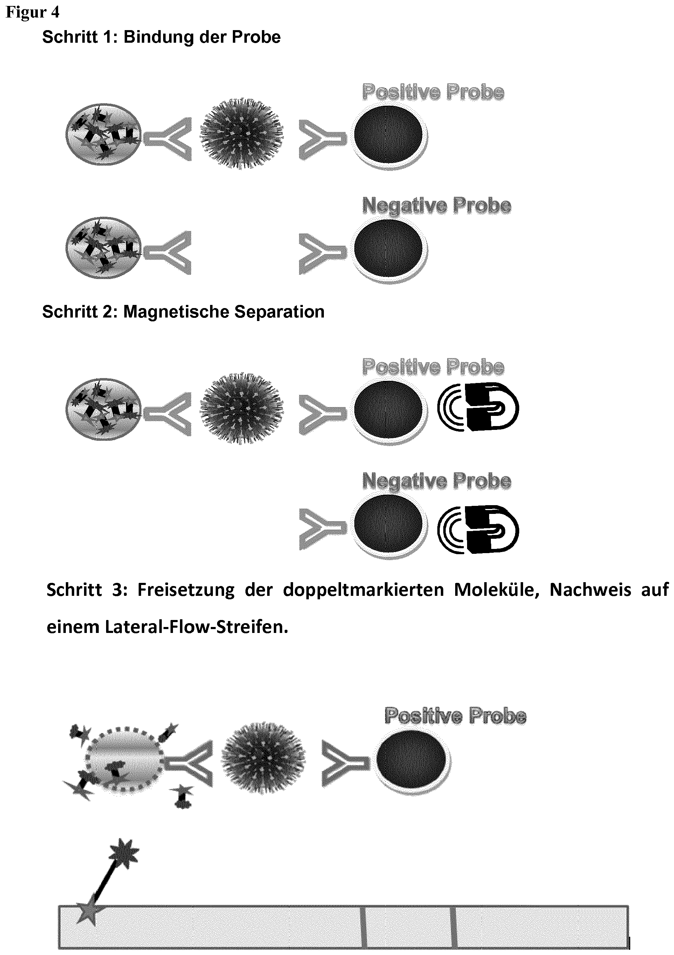

Die Partikel wurden zweimal gewaschen und nachfolgend chromatographisch gereinigt. Das Auftragen des Partikelüberstandes auf einen Lateral Flow Streifen mit den Bindungsdomänen für FITC und Dioxigenin sollte eine Information über die Reinheit der Partikel vom ungebundenen doppeltmarkierten Nachweisoligonukleotid liefern. Auf der Figur 4 (Streifen A) ist der Streifen dargestellt. Das Signal auf dem Streifen zeigt, dass die Partikel trotz des mehrmaligen Waschens und einer chromatographischen Reinigung nicht frei von den ungebundenen Nachweismolekülen sind. Die Partikel wurden weiter mit großen Volumina an Tris-BSA-Puffer gewaschen, bis der Überstand kein positives Resultat auf dem Lateral Flow Streifen zeigte (Figur 4; Streifen B).

Nachfolgend musste gezeigt werden, dass die Nachweispartikel trotz des mehrmaligen Waschens funktional bleiben, also dass das doppeltmarkierte Oligonukleotid immer noch an das kovalent am Partikel gebundene Oligonukleotid hybridisiert ist. Dafür wurden die gereinigten Nachweispartikel auf 70°C erhitzt, um die Hybridisierung zwischen den beiden Oligonukleotiden zu lösen. Der Überstand nach Erhitzung wurde wiederum auf einen Lateral Flow Streifen aufgetragen. In diesem Fall zeigte der Lateral Flow Streifen ein positives Resultat (Figur 4; Streifen C). Dies beweist, dass die Nachweipartikel einerseits sauber sind und keine freien Nachweismoleküle mehr beinhalten (was zu falschpositiven Resultaten führt), andererseits funktional aktiv für den weiteren Nachweis sind.

Das Experiment demonstrierte darüber hinaus die Wichtigkeit der doppeltmarkierten Nachweismoleküle für die Prozessüberwachung bei der Herstellung der Nachweismoleküle.

### Beispiel 2:

### Vakziniavirusnachweis mittels funktionalisierter Polymer-Detektionspartikeln mit eingeschlossenen Nachweismolekülen

Als Nachweispartikel dienen dabei Nanokapseln, welche die doppeltmarkierten Nachweismoleküle nicht auf der Oberfläche präsentieren, sondern diese eingeschlossen haben. Diese Polyester-Nanopartikeln werden durch eine direkte Miniemulsion unter Verwendung des Monomers 5,6-Benzo-2-methylen-1,3-dioxepan (BMDO) hergestellt und danach durch Carbodiimidazolkopplungen nachfunktionalisiert. Diese Herstellung erlaubt neben der Verkapselung der DNA-Moleküle auch eine Antikörperanbindung an der Oberfläche der Nanopartikel. Die Nachweispartikel unterscheiden sich von den anderen beschrieben Nachweispartikeln nur dadurch, dass die Nachweismoleküle jetzt im Inneren der Partikel sind, und nicht an der Oberfläche. An der Oberfläche befindet sich jetzt nur noch der Antikörper.
Die Nachweispartikeln und die Separationspartikel werden mit anti-Vakzinia-Virus Antikörpern funktionalisiert. Der Nachweis der Vakziniaviren erfolgt wie nachfolgend beschrieben. Detektionspartikel und Nachweispartikel werden in die Probe mit den enthaltenen Vakziniaviren gegeben:
Schritt 1: Bildung eines Komplexes aus Separationspartikel-Virus-Nachweispartikel. Es entsteht ein Komplex zwischen den Nachweispartikeln und den Separationspartikeln und den Vakziniaviren. Im Falle einer targetnegativen Probe erfolgt keine Komplexbildung und die Nachweispartikel sowie Separationspartikel bleiben voneinander getrennt.
Schritt 2: Magnetische Separation. Im Falle einer positiven Probe wird der gebildete Komplex (Separationspartikel-Virus-Nachweispartikel) via Magnetseparation von der Probenlösung getrennt und nachfolgend zweimal mit PBS gewaschen und nach einer erneuten Separation kurz getrocknet.
Schritt 3:Freisetzung der doppeltmarkierten Moleküle, Nachweis auf einem Lateral-Flow-Streifen. Eine 15 minütige Inkubation bei 85°C erzeugt einen Dampfdruck innerhalb der Nachweispartikel. Dieser bewirkt das Platzen der Partikelwände und die Freisetzung der doppeltmarkierten Moleküle. Nach Zugabe von Wasser und einem kurzen Vortexschritt wird der Partikelüberstand auf einen Lateral-Flow-Streifen aufgetragen. Dort erfolgt der Nachweis der doppeltmarkierten DNA Molekülen und damit der Nachweis des Vorliegens von Vakziniaviren in der Probe.

Das Ausführungsbeispiel ist in Figur 4 noch einmal schematisch dargestellt

### Beispiel 3:

### Nachweis einer geringen Menge an Gelbfiebervirus mit Hilfe der erfindungsgemäß hergestellten Partikeln.

Als Nachweispartikel wurden die im Ausführungsbeispiel 1 hergestellten Partikel verwendet. Als Separationspartikel wurden COOH-funktionalisierte Dextran-Eisenoxid-Partikel verwendet. Diese Partikel wurden mit dem PolyLink- Protein Coupling Kit für COOH Mikropartikel nach Vorschriften des Kits mit dem gleichen Antikörper wie auch die Nachweispartikel gekoppelt. Als Analyten wurden Gelbfiber Viren aus einer Viruskultur verwendet. Die Konzentration der Viren betrug 1000 PFU/ ml.

### Reaktionsansatz:

| | **Positive Probe** | **Negative Probe** |
|---|---|---|
| Viruskultur | 100 µl ∼100 PFU | - |
| 1xPBS | 100 µl | 200 µl |
| Nachweispartikel | 5 µl | 5 µl |
| Separationspartikel | 5 µl | 5 µl |

### Arbeitsschritte:

1. Der Reaktionsansatz wurde in 2 ml- Reaktionsgefäßen für 10 min bei Raumtemperatur inkubiert
2. Die Reaktionsgefäße wurden in eine Magnetfalle gestellt.
3. Der Probenüberstand wurde verworfen.
4. Die Proben wurden in der Magnetfalle 2x gewaschen.
5. Nach der Entfernung der Waschlösung wurde zu jeder Probe 100 µl Wasser zugefügt.
6. Die Proben wurden aus der Magnetfalle entnommen und 10 min bei 70°C inkubiert.
7. Die Proben wurden erneut in eine Magnetfalle gestellt.
8. 5µl des Überstandes wurden auf einen Lateral Flow Streifen zur Detektion der freigesetzten Nachweispartikel aufgetragen.

Gesamtzeitaufwand incl. Inkubationszeiten max. 30 min.

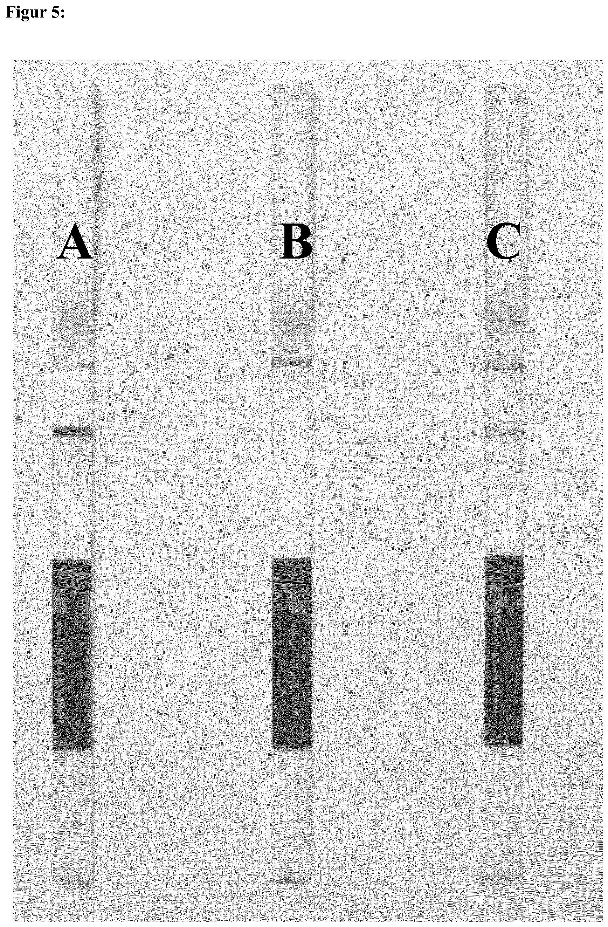

Die Ergebnisse des Nachweises für eine positive und eine negative Probe sind auf der Figur 5 dargestellt. Wie das Ergebnis zeigt, konnte der direkte Nachweis von Gelbfieber Viren erfolgreich durchgeführt werden. Dabei zeichnet sich das Verfahren durch seine Einfachheit (es wird kein Gerät benötigt) sowie durch seine hohe Sensitivität und schnelle Durchführung aus.

### Beispiel 4:

### Vergleich der Nachweisensitivität eines Lateral Flow Tests mit einer Fluoreszenzvermessung

Für das Experiment wurden FAM- Biotin gelabelte Nachweismoleküle verwenden, hierbei kann die FAM- Markierung sowohl als Immunogen für eine Antikörperbindung eines Lateral Flow Streifens als auch als ein fluoreszierendes Stoff für eine Fluoreszenzmessung dienen.

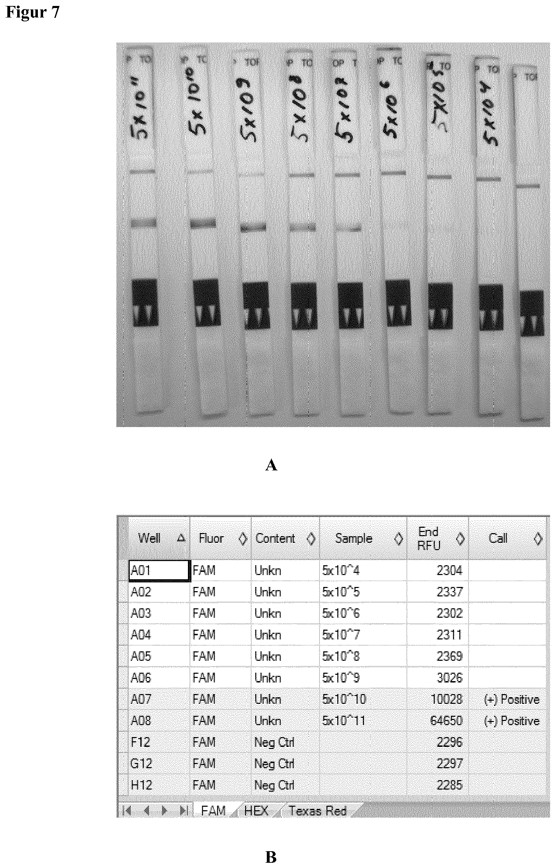

Die Nachweismoleküle wurden in einer Verdünnungsreihe gemessen. (s. Tabelle und Fig. 7A und B). Die Fluoreszenzmessung erfolgte mit einem real time PCR- Gerät als Endpunktmessung.

| **Anzahl Nachweismoleküle** | **Lateral Flow Streifen** | **Endpunkt Fluoreszenzmessung** |
|---|---|---|
| **5x10¹¹** | **positiv** | **positiv** |
| **5x10¹⁰** | **positiv** | **positiv** |
| **5x10⁹** | **positiv** | **fraglich** |
| **5x10⁸** | **positiv** | **negativ** |
| **5x10⁷** | **positiv** | **negativ** |
| **5x10⁶** | **positiv** | **negativ** |
| **5x10⁵** | **positiv** | **negativ** |
| **5x10⁴** | **negativ** | **negativ** |

Das Experiment hat gezeigt, dass sowohl eine gerätefreie Detektion als auch eine auf Fluoreszenmessung basierte Detektion der Nachweismoleküle möglich ist. Darüber hinaus konnte demonstriert werden, dass die laborgängigen Fluoreszenzmessgeräte eine ca. 10000-fach geringere Sensitivität aufweisen, als ein gerätefreier Lateral Flow Test. Also müssen die Proben bei anderen "Biobarcode- basierten" Nachweisverfahren, in denen die Fluoreszenz der Nachweismoleküle gemessen wird, mit sehr teurer und empfindlicher Technik arbeiten, um die gleiche Sensitivität zu erreichen.

### Beispiel 5:

### Ein Beispiel für eine Lateral Flow basierte Multiplexdetektion der Nachweispartikel

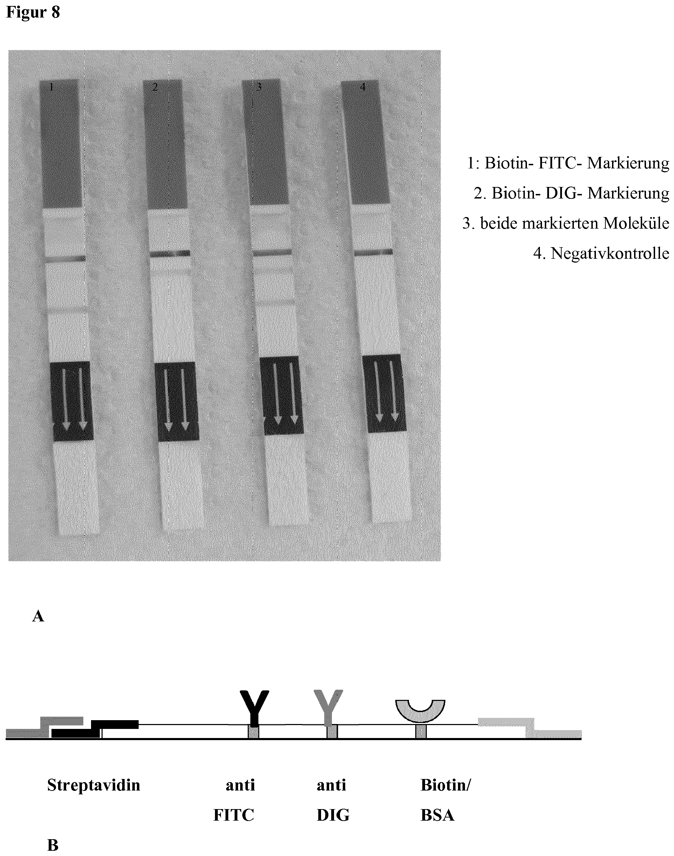

In diesem Experiment wurden die Nachweispartikel mit unterschiedlichen Labeln markiert Die Ergebnisse des Experiments und eine schematische Darstellung eines Multiplex- Lateral Flow Streifens sind auf der Fig. 8A und B dargestellt.

### Erläuterung zu den Figuren

Figur 1 zeigt das Prinzip des Nachweises für Nukleinsäuren und Antigene:
1. Zusammenbringen von Analyt, Nachweispartikeln und Separationspartikeln.
2. Magnetische Separation und Abwaschen der nichtgebundenen Partikel und Moleküle. Anschließende Ablösung der Nachweispartikel.
3. Direkter Nachweis der doppeltmarkierten Nachweispartikel auf einem Lateral Flow Streifen.
4. Kaskadenartige Verstärkungsreaktion durch ein zweites Nachweiszyklus.

In Figur 2 ist eine schematische Darstellung der Bindung eines Nukleinsäureanalyten an die Nachweispartikel zu erkennen. Eine schematische Darstellung des funktionalisierten Nachweispartikels zeigt Figur 3. Die abgebildeten Oligonukleotidkomplexe und Antikörper sind mehrfach auf der Oberfläche des Partikels gebunden. Figur 4 ist eine Darstellung gemäß Beispiel 2.

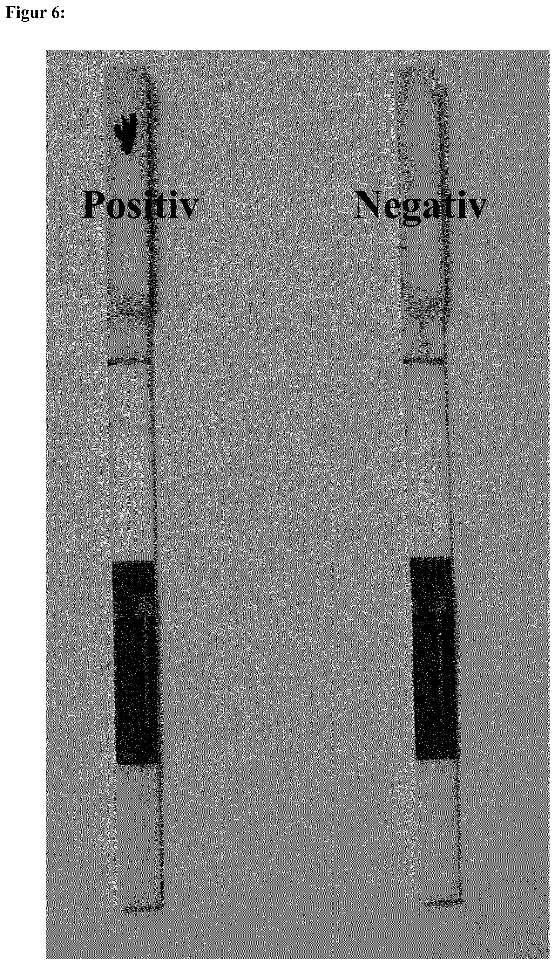

Ergebnisse des Lateral Flow Tests im Prozess der Herstellung der funktionalisierten Nachweipartikel sind in Figur 5 abgebildet. Figur 6 zeigt den Nachweis einer geringen Menge an Gelbfiebervirus. Vergleich der Detektionssensitivität eines Lateral Flow Streifens (A) mit einer Fluoreszenzmessung (B) ist Gegenstand von Figur 7. Figur 8 zeigt das Beispiel einer Multiplexdetektion der Nachweispartikel.

## Patentansprüche

1. Verfahren zur Detektion von diversen Analyten, **gekennzeichnet durch** folgende Schritte:
a) Bereitstellen von Separationspartikeln, die an ihrer Oberfläche einerseits Mittel zur Anbindung des nachzuweisenden Analyten und anderseits Mittel zur Separation des an die Partikel angebundenen Analyten enthalten
b) Bereitstellen von Nachweispartikeln, die an ihrer Oberfläche einerseits Mittel zur Anbindung des nachzuweisenden Analyten aufweisen und andererseits Mittel an ihrer Oberfläche oder in sich eingeschlossen enthalten, die nach deren Ablösung oder Freisetzung von den Partikeln in der Lage sind, durch ihre Markierung ein Signal zu erzeugen, welches dem Nachweis des Analyten dient
c) Zusammenführen von Analyt, Separationspartikeln und Nachweispartikeln
d) Abtrennen und Waschen der über den Analyten gebundenen Nachweispartikel mittels der Separationspartikel
e) Freisetzung der Mittel, die zum Nachweis des Analyten dienen,
**dadurch gekennzeichnet, dass** der Nachweis auf einem Lateral Flow Streifen erfolgt und dass die Mittel, die zum Nachweis des Analyten dienen, reversibel an die Nachweispartikel gekoppelt sind und dass die Nachweismoleküle gleichzeitig dem Nachweis des Analyten und der Detektion dienen.

2. Verfahren nach Anspruch 1, **dadurch gekennzeichnet, dass** als Mittel zum Nachweis des Analyten markierbare (Nachweis-) Moleküle dienen, die entweder an Nukleinsäuren reversibel gekoppelt sind, die ihrerseits irreversibel, vorzugsweise kovalent, an eine funktionalisierte Oberfläche der Nachweispartikel gebunden sind oder in die Nachweispartikel eingeschlossen sind..

3. Verfahren nach Anspruch 1 oder 2, **dadurch gekennzeichnet, dass** als Nachweispartikel silikatische Partikel verwendet werden, an denen die Nachweismoleküle via Spacer gebunden sind oder dass magnetische Partikel als Separationspartikel mit magnetischem Kern, aber silikatischer Hülle, eingesetzt werden.

4. Verfahren nach einem der Ansprüche 1 bis 3, **dadurch gekennzeichnet, dass** als Nachweispartikel solche Partikel eingesetzt werden, die keinen spezifischen Wechselwirkungspartner auf der Oberfläche tragen und unspezifisch Analyten binden oder dass als Nachweispartikel solche Partikel eingesetzt werden, die mehrere Substanzen binden können.

5. Verfahren nach Anspruch 1 oder 2, **dadurch gekennzeichnet, dass** es sich bei der funktionalisierten Oberfläche der Nachweispartikel um eine COOH-funktionalisierte Oberfläche handelt, an die NH₂-gekoppelte Nukleinsäuren irreversibel gebunden sind.

6. Verfahren nach einem der Ansprüche 1 bis 5, **dadurch gekennzeichnet, dass** die markierten Nachweismoleküle mindestens eine, vorzugsweise zwei Markierungen aufweisen, die einen direkten Nachweis von Nukleinsäuremolekülen erlauben.

7. Verfahren nach Anspruch 6, **dadurch gekennzeichnet, dass** als Markierungen FITC, Biotin, Digoxigenin oder Tamra™ (5-carboxytetramethylrhodamin) fungieren.

8. Verfahren nach einem der Ansprüche 1 bis 7, **dadurch gekennzeichnet, dass** als Analyten sowohl Immunogene als auch Nukleinsäuresequenzen dienen können.

9. Verfahren nach einem der Ansprüche 1 bis 8, **dadurch gekennzeichnet, dass** bei der Detektion von Nukleinsäuren als Analyt die markierten Nachweismoleküle gleichzeitig als Mittel zur Anbindung des nachzuweisenden Analyten an die Nachweispartikel dient und auf ein gesondertes Mittel zur Anbindung des nachzuweisenden Analyten an die Nachweispartikel verzichtet werden kann.

10. Verfahren nach einem der Ansprüche 1 bis 9, **dadurch gekennzeichnet, dass** die markierten Nachweismoleküle selbst als Analyt für eine Signalverstärkung dienen können.

11. Verfahren nach einem der Ansprüche 1 bis 10, **dadurch gekennzeichnet, dass** die Separationspartikel magnetisch sind oder sie eine Funktionalität aufweisen, welche einen Separationsprozess ermöglicht oder
dass an Stelle der Partikel eine feste Phase mit derselben Funktionalität wie die Separationspartikel verwendet wird.

12. Verfahren nach einem der Ansprüche 1 bis 11, **dadurch gekennzeichnet, dass** die Größe der Partikel zwischen 1 nm und 10 µm liegt.

13. Verfahren nach einem der Ansprüche 1 bis 12, **dadurch gekennzeichnet, dass** die Nachweispartikel aus Gold, Latex, Polymeren oder Fullerenen bestehen.

## Claims

1. A method for detecting various analytes, **characterised by** the following steps:
a) Providing separation particles containing on their surface on the one hand means of binding the analyte to be identified and on the other hand means of separating the analyte bound to the particles
b) Providing identification particles having on their surface on the one hand means of binding the analyte to be identified and on the other hand containing on their surface or enclosed therein means, which are capable, after they have been detached or released from the particles, by virtue of their labeling of generating a signal which serves for identification of the analyte
c) Combining analyte, separation particles and identification particles
d) Removing and washing the identification particles bound via the analyte by means of the separation particles
e) Releasing the means which serve to identify the analyte,
**characterized in that** the identification occurs on a lateral flow strip, and that the means serving for identification of the analyte, are reversibly coupled to the identification particles, and that the identification molecules simultaneously serve for identification of the analyte, and for detection.

2. The method according to claim 1, **characterized in that** (identification) molecules that can be labeled serve as means for identification of the analyte, which molecules are either reversibly coupled to nucleic acids, which in turn are irreversibly, preferably covalently, bound to a functionalized surface of the identification particles or are enclosed in the identification particles.

3. The method according to claim 1 or 2, **characterised in that** siliceous particles are used as identification particles to which the identification molecules are bound via spacers or that magnetic particles are used as separation particles with magnetic core but a siliceous shell.

4. The method according to any one of claims 1 to 3, **characterized in that** as identification particles such particles are used, which do not carry any specific interaction partner on the surface, and unspecifically bind analytes or that as identification particles such particles are used which can bind several substances.

5. The method according to claim 1 or 2, **characterised in that** the functionalized surface of the identification particles is a COOH functionalized surface to which NH₂ coupled nucleic acids are irreversibly bound.

6. The method according to any one of claims 1 to 5, **characterized in that** the labeled identification molecules comprise at least one, preferably two, labelings permitting direct identification of nucleic acid molecules.

7. The method according to claim 6, **characterized in that** FITC, biotin, digoxigenin or Tamra™ (5-carboxytetramethylrhodamine) act as a labeling.

8. The method according to any one of claims 1 to 7, **characterized in that** not only immuno"-gens but also nucleic acid sequences can serve as analytes.

9. The method according to any one of claims 1 to 8, **characterized in that** in the detection of nucleic acids as an analyte the labeled identification molecules simultaneously serve as a means for binding the analyte to be identified to the identification particles, and a separate means for binding the analyte to be identified to the identification particles can be waived.

10. The method according to any one of claims 1 to 9, **characterized in that** the labeled identification molecules themselves can serve as an analyte for a signal amplification.

11. The method according to any one of claims 1 to 10, **characterized in that** the separation particles are magnetic or comprise a functionality permitting a separation process or that instead of the particles a solid phase with the same functionality as the separation particles is used.

12. The method according to any one of claims 1 to 11, **characterized in that** the size of the particles ranges between 1 nm and 10 µm.

13. The method according to any one of claims 1 to 12, **characterized in that** the identification particles are comprised of gold, latex, polymers or fullerenes.

## Revendications

1. Procédé pour détecter des analytes différents, **caractérisé par** les étapes suivantes:
a) fournir des particules de séparation contenant à leur surface d'une part des moyens pour la liaison de l'analyte à identifier et d'autre part des moyens pour la séparation de l'analyte lié aux particules
b) fournir des particules d'identification comportant à leur surface d'une part des moyens pour la liaison de l'analyte à identifier et d'autre part contentant des moyens à leur surface ou enfermés en soi qui après leur détachement ou libération des particules sont capables de générer un signal par leur marquage qui sert à identifier l'analyte
c) combiner l'analyte, les particules de séparation et les particules d'identification
d) enlever et laver les particules d'identification liées par l'analyte au moyen des particules de séparation
e) libération des moyens qui servent à identifier l'analyte,
**caractérisé en ce que** l'identification est fait sur une bande d'écoulement latéral et que les moyens qui servent à identifier l'analyte sont couplés réversiblement aux particules d'identification et que les molécules d'identification servent simultanément à identifier l'analyte et à la détection.

2. Procédé selon la revendication 1, **caractérisé en ce que** des molécules (d'identification) qui peuvent être marquées servent comme moyen pour l'identification de l'analyte, les molécules étant couplées réversiblement aux acides nucléiques qui sont pour leur part couplés irréversiblement, de préférence de manière covalente, liés à une surface fonctionnalisée des particules d'identification ou étant enfermées dans les particules d'identification.

3. Procédé selon la revendication 1 ou 2, **caractérisé en ce que** des particules siliceuses sont utilisées comme particules d'identification auxquelles les molécules d'identification sont liées via des espaceurs ou que des particules magnétiques sont utilisées comme particules de séparation avec un noyau magnétique mais une enveloppe siliceuse.

4. Procédé selon l'une quelconque des revendications 1 à 3, **caractérisé en ce que** comme particules d'identification des particules sont utilisées qui ne portent pas un partenaire spécifique d'interaction à la surface et lient des analytes de manière non spécifique ou
**que** des particules sont utilisées comme particules d'identification qui sont capables de lier plusieurs substances.

5. Procédé selon la revendication 1 ou 2, **caractérisé en ce que** la surface fonctionnalisée des particules d'identification est une surface fonctionnalisée COOH à laquelle des acides nucléiques NH₂ couplée sont liés irréversiblement.

6. Procédé selon l'une quelconque des revendications 1 à 5, **caractérisé en ce que** les molécules d'identification marquées comportent au moins un marquage, de préférence deux marquages, permettant une identification directe des molécules d'acides nucléiques.

7. Procédé selon la revendication 6, **caractérisé en ce que** le FITC, la biotine, la digoxigénine ou Tamra™ (5-carboxytétraméthylerhodamine) agissent comme marquages.

8. Procédé selon l'une quelconque des revendications 1 à 7, **caractérisé en ce que** non seulement des immunogènes mais aussi des séquences d'acides nucléiques peuvent servir comme analytes.

9. Procédé selon l'une quelconque des revendications 1 à 8, **caractérisé en ce que** lors de la détection des acides nucléiques comme analyte les molécules d'identification marquées simultanément servent comme moyen pour la liaison de l'analyte à identifier aux particules d'identification et que l'on peut renoncer à un moyen séparé pour la liaison de l'analyte à identifier aux particules d'identification.

10. Procédé selon l'une quelconque des revendications 1 à 9, **caractérisé en ce que** les molécules d'identification marquées peuvent servir elles-mêmes comme analyte pour une amplification du signal.

11. Procédé selon l'une quelconque des revendications 1 à 10, **caractérisé en ce que** les particules de séparation sont magnétiques ou qu'elles comprennent une fonctionnalité permettant un procédé de séparation ou qu'au lieu des particules une phase solide avec la même fonctionnalité comme les particules de séparation est utilisée.

12. Procédé selon l'une quelconque des revendications 1 à 11, **caractérisé en ce que** la taille des particules est entre 1 nm et 10 µm.

13. Procédé selon l'une quelconque des revendications 1 à 12, **caractérisé en ce que** les particules d'identification sont en or, en latex, en polymères ou en fullerènes.
